# EUROPEAN PATENT SPECIFICATION

(11) **EP 4 302 733 B1**
(45) Date of publication and mention of the grant of the patent: **10.12.2025**
(21) Application number: 23183020.9
(22) Date of filing: 03.07.2023
(51) Int. Cl.: A61F 5/44, A61F 5/445

(54) **OSTOMY DEVICE FOR DIGESTIVE TRACTS**
OSTOMIEVORRICHTUNG FÜR VERDAUUNGSTRAKTE
DISPOSITIF DE STOMIE POUR VOIES DIGESTIVES

(30) Priority: 04.07.2022 CN 202210786510
(43) Date of publication of application: 10.01.2024
(73) Proprietor: HONEST MEDICAL CHINA CO., LTD., ZhuHai 519031 (CN)
(72) Inventor: LEI, Chi-Weng, ZhuHai, 519031 (CN); LI, Zhao-Yin, ZhuHai, 519031 (CN); NI, Jui-Chung, ZhuHai, 519031 (CN); LIN, Huang-Chen, ZhuHai, 519031 (CN)
(74) Representative: Viering, Jentschura & Partner mbB Patent- und Rechtsanwälte

(56) References cited:
- WO-A1-2022/079708
- US-A1- 2012 136 324
- US-A1- 2013 079 737

## Description

### FIELD OF THE INVENTION

The present invention relates to an ostomy device for digestive tracts, and more particularly to an ostomy device having an ostomy body provided with sensor units for sensing the conditions of the digestive tracts or a stoma regularly or at any time.

### BACKGROUND OF THE INVENTION

According to the conditions (such as dysphagia) of a patient, the doctor may create a stoma in the digestive tract after evaluation, and a fistula is directly inserted from the outside of the body to pass through the stoma into the digestive tract to form an ostomy device, such that the caregiver can directly administer medicine or perform tube-feeding through the fistula.

Because the stoma directly penetrates the digestive tract from the body surface, it is usually covered with medical dressings such as gauze. The gauze needs to be replaced with a new one frequently, so as to prevent a leak of acidic or inflammatory substances in the digestive tract because the leak may damage the tissue or skin near the stoma. Besides, regular check-ups are required to confirm the condition of the ostomy device by medical personnel.

Chinese Patent Publication No. CN114080204 titled "LEAK DETECTION SYSTEM FOR OSTOMY APPLIANCES", US Patent Application Early Publication No. US20220031495 titled "OSTOMY MONITORING SYSTEM AND METHOD" and US Patent Application Early Publication No. US20220079803 titled "A SENSOR PATCH FOR AN OSTOMY APPLIANCE" disclose automatic detection of the condition of an ostomy device. In the above-mentioned patent/applications, detectors are disposed on the body surface for detecting the condition of the stoma, such as whether the digestive juice leaks, or for detecting the temperature near the stoma.

However, although the detectors are disposed on the body surface for detecting a leak of digestive juice, the digestive juice has already leaked from the digestive tract for a period of time. It is unable to deal with the problem of digestive juice leak in real time. Besides, the detector is unable to detect the wound on the cut surface of the stoma or the condition inside the digestive tract.

Chinese Patent No. CN202110962922.0 discloses "A Multifunctional Water Bladder Gastrostomy Tube". As disclosed in this patent, a water bladder 2 is provided to support the inside of a stoma of the human body for retaining the gastrostomy tube. The gastrostomy tube has a water injection passage 23 and a monitoring passage 22 therein. The water injection passage 23 is configured for injecting water into the water bladder 2 so that the water bladder 2 expands and supports the inside of the stoma. A pressure detector 221 extends into the water bladder 2 for detecting the pressure of the water bladder 2. Then, the pressure value is output to a visualization module 4 outside the gastrostomy tube through the monitoring passage 22. As shown in FIG. 5 and FIG. 6 of this patent, the pressure detector 221 can protrude from the gastrostomy tube and extend into the stomach when in use.

The pressure detector 221 of this patent can sense the pressure in the water bladder 2 and the stomach, but it cannot detect the condition of the passage in the gastrostomy tube or the condition of the tube wall in contact with the periphery of the stoma. Therefore, it is impossible to know whether gastric juice leaks through the periphery of the stoma or from the inner passage of the gastrostomy tube.

US 2013/079737 A1 discloses an ostomy device for digestive tracts according to the preamble of claim 1.

Further ostomy devices are known from US 2012/136324 A1 and WO 2022/079708 A1.

### SUMMARY OF THE INVENTION

In view of the drawbacks of the prior art, the primary object of the present invention is to an ostomy device for digestive tracts. The present invention provides an ostomy device according to claim 1. The dependent claims show further embodiments of the said ostomy device. The ostomy device comprises an ostomy body, an outer retaining plate, an inner retaining member, at least one sensor unit, a processing unit, and a power supply unit. The ostomy body includes a first end and a second end. The ostomy body further includes an outer surface, an inner surface, and an ostomy hole passing through the ostomy body. The outer retaining plate includes an outer contact surface. The outer retaining plate is disposed close to the first end. The inner retaining member includes an inner contact surface. The inner retaining member is disposed on the second end. The sensor unit includes a sensing surface. The sensor unit is disposed on at least one of the outer surface, the inner surface, the outer contact surface and the inner contact surface. The sensing surface is exposed on the outer surface, the inner surface, the outer contact surface or the inner contact surface. The processing unit is in signal communication with the sensor unit for receiving sensor information of the sensor unit. The power supply unit is electrically connected to the sensor unit and the processing unit.

In an embodiment of the present invention, the sensor unit is annularly arranged on the outer contact surface.

In an embodiment of the present invention, the sensor unit is annularly arranged on the inner contact surface.

In an embodiment of the present invention the sensor unit is annularly arranged on the outer surface.

In an embodiment of the present invention, the sensor unit is annularly arranged on the inner surface.

Furthermore, the sensor unit is one of a blood glucose sensor, a pH sensor, a tension sensor, a blood oxygen sensor, a temperature sensor and a flow rate sensor.

Furthermore, the ostomy device further comprises an ultrasonic unit on the ostomy body. The ultrasonic unit is electrically connected to the power supply unit and is in signal communication with the processing unit.

Furthermore, the ostomy device further comprises a wireless unit. The wireless unit is in signal communication with the processing unit. The wireless unit is electrically connected to the power supply unit. The wireless unit and the processing unit are disposed on the ostomy body. The processing unit receives a sensor signal from the sensor unit and transmits the sensor signal through the wireless unit.

Furthermore, the ostomy device further comprises a warning unit. The warning unit is in signal communication with the processing unit. The warning unit is electrically connected to the power supply unit.

Furthermore, the processing unit and the power supply unit are disposed on an outside of the ostomy body. The power supply unit is connected to the sensor unit with a wire. The processing unit is connected to the sensor unit through a signal wire.

Furthermore, the sensing surface is flush with or recessed in the outer contact surface, the inner surface, the outer surface, or the inner contact surface; or the sensing surface protrudes from the outer contact surface.

Furthermore, the sensor unit is disposed in an inner groove. Furthermore, an extension axis of the inner groove is perpendicular or inclined to the outer contact surface, the inner surface, the outer surface, or the inner contact surface. Furthermore, the inner groove is annular.

Furthermore, the sensor unit is annular.

According to the above technical features, the present invention can achieve the following effects:
1. In the present invention, the sensor units are disposed on the outer surface and the inner surface of the ostomy body, the outer contact surface and the inner contact surface for detecting the conditions of the inside of the digestive tract, the inside of the ostomy hole, the wound on the cut surface of the stoma and the body surface. Especially, when digestive juices or inflammatory substances leak from the digestive tract, the sensor units can detect the conditions in real time for the caregiver to take care of the stoma.
2. The sensor unit may be disposed in the inner groove of the outer surface of the ostomy body. When a little leak of digestive juice is not detected by the sensor unit on the outer surface of the ostomy body, the digestive juice accumulated in the inner groove can be detected by the sensor unit in the inner groove, so that the detection of digestive juice leak is more real-time and accurate. Furthermore, the inner groove is ring-shaped, so that a leak from all directions can be detected.
3. The sensor unit may be different sensors, such as a blood glucose sensor, a pH value sensor, a tension sensor, a blood oxygen sensor, a temperature sensor, a flow rate sensor, etc. for sensing various conditions of the stoma.
4. The ultrasonic unit is configured to detect the suturing condition of the ostomy operation, whether the wound is purulent, and whether there is blood clot remaining in the stoma, and the like.
5. The sensor unit may be ring-shaped, which is beneficial to sense the conditions of the stoma of the digestive tract in all directions.

### BRIEF DESCRIPTION OF THE DRAWINGS

FIG. 1 is a perspective view of the ostomy device according to a first embodiment of the present invention;
FIG. 2 is another perspective view of the ostomy device according to the first embodiment of the present invention;
FIG. 3 is a cross-sectional view of the ostomy device according to the first embodiment of the present invention;
FIG. 4 is a block diagram of the connection relationship of the electronic components in the ostomy device according to the first embodiment of the present invention;
FIG. 5 is a cross-sectional view of the ostomy device according to the first embodiment of the present invention when applied to the stoma of the digestive tract;
FIG. 6 is a schematic view of the ostomy device according to a second embodiment of the present invention, wherein the electronic components are disposed on the outside of the ostomy device;
FIG. 7 is a schematic view of the ostomy device according to a third embodiment of the present invention, wherein the ultrasonic device is disposed on the ostomy body;
FIG. 8 is a block diagram of the connection relationship of the electronic components in the ostomy device according to the third embodiment of the present invention;
FIG. 9 is a schematic view of the ostomy device according to a fourth embodiment of the present invention, wherein the sensing surface of the sensor unit protrudes from the outer contact surface;
FIG. 10 is a cross-sectional view of the ostomy device according to a fifth embodiment of the present invention;
FIG. 11 is a cross-sectional view of FIG. 10, illustrating that the sensor unit is disposed in the annular inner groove; and
FIG. 12 is a cross-sectional view of FIG. 10, illustrating that the annular sensor unit is disposed on the outer surface of the ostomy body.

### DETAILED DESCRIPTION OF THE PREFERRED EMBODIMENTS

Embodiments of the present invention will now be described, by way of example only, with reference to the accompanying drawings.

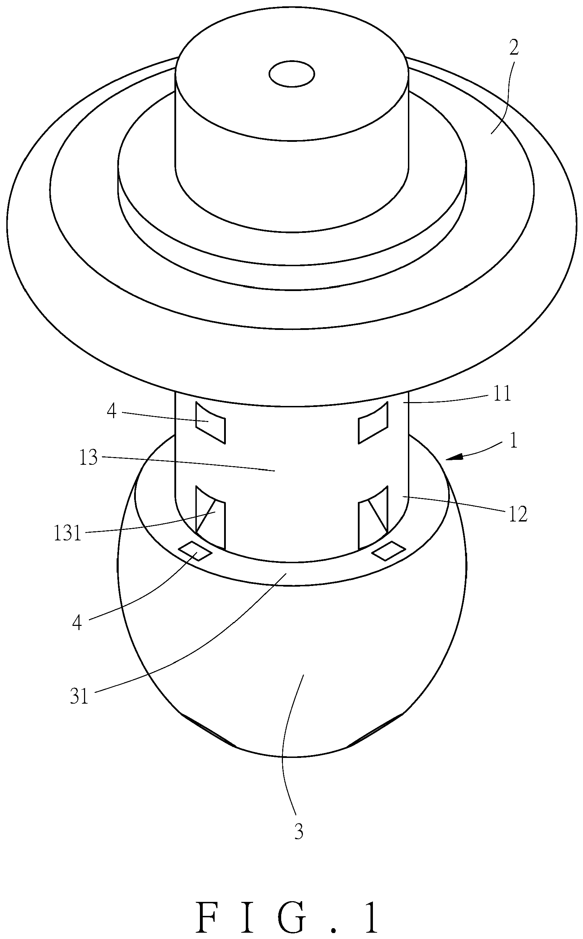

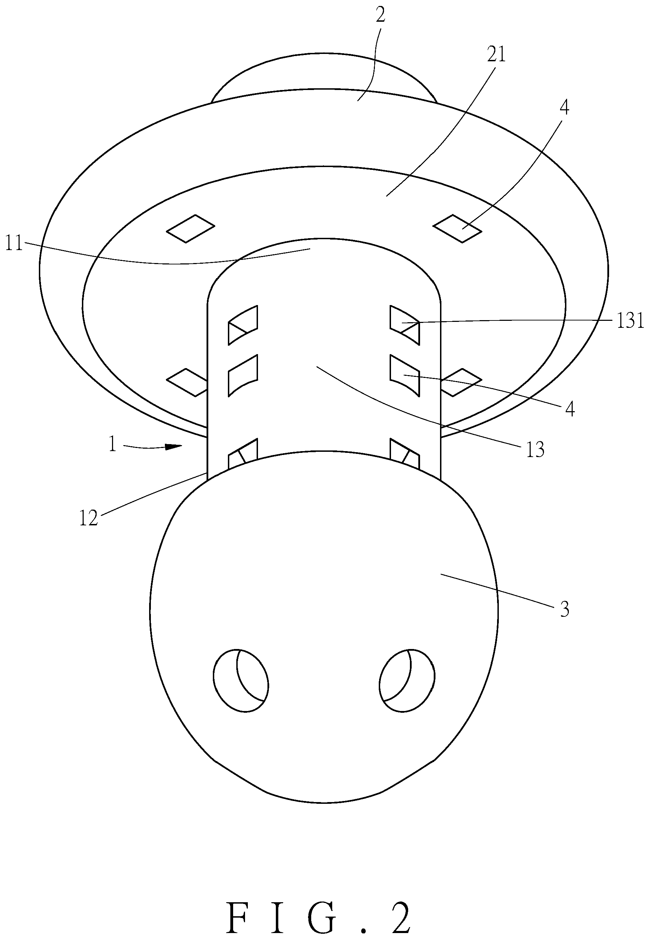

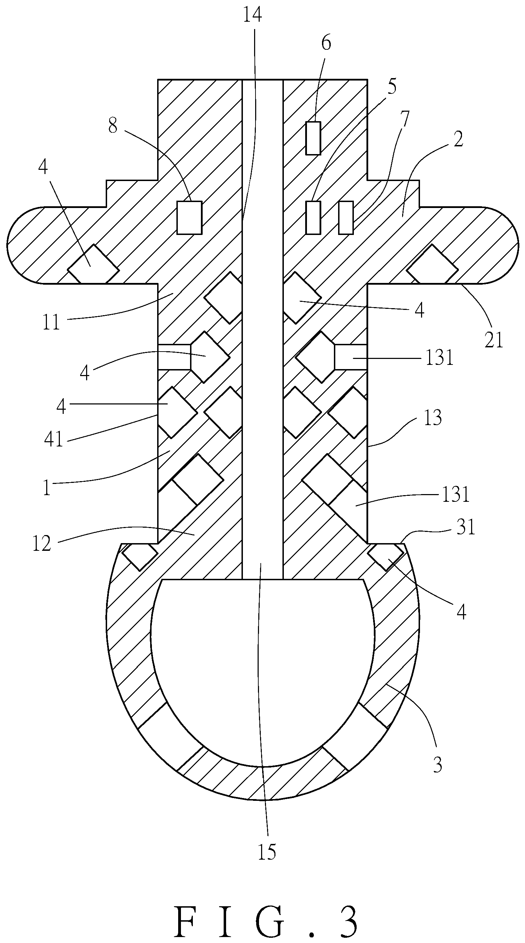

A first embodiment of the present invention is shown in FIG. 1, FIG. 2, and FIG. 3. The ostomy device for digestive tracts of this embodiment comprises an ostomy body 1, an outer retaining plate 2, and an inner retaining member 3. The ostomy body 1 includes a first end 11 and a second end 12. The ostomy body 1 further includes an outer surface 13, an inner surface 14, and an ostomy hole 15 passing through the ostomy body 1. The outer retaining plate 2 includes an outer contact surface 21. The outer retaining plate 2 is disposed close to the first end 11. The inner retaining member 3 includes an inner contact surface 31. The inner retaining member 3 is disposed on the second end 12.

At least one sensor unit 4 is disposed on at least one of the outer surface 13, the inner surface 14, the outer contact surface 21 and the inner contact surface 31. In this embodiment, a plurality of sensor units 4 are provided on the outer surface 13, the inner surface 14, the outer contact surface 21 and the inner contact surface 31, respectively. The sensor units 4 are annularly arranged on the outer surface 13, the inner surface 14, the outer contact surface 21 and the inner contact surface 31, respectively. The sensor unit 4 includes a sensing surface 41. The sensing surface 41 is exposed on the outer surface 13, the inner surface 14, the outer contact surface 21 and the inner contact surface 31. Specifically, in this embodiment, the sensing surface 41 is flush with the inner surface 14, the outer contact surface 21 and the inner contact surface 31. One part of the sensor units 4 located on the outer surface 13 is flush with the outer surface 13, and the other part of the sensor units 4 is recessed in the outer surface 13. For example, the outer surface 13 is formed with inner grooves 131 that are arranged annularly. The sensor units 4 are disposed in the inner grooves 131, respectively. An extension axis of the inner groove 131 may be perpendicular or inclined to the outer surface 13.

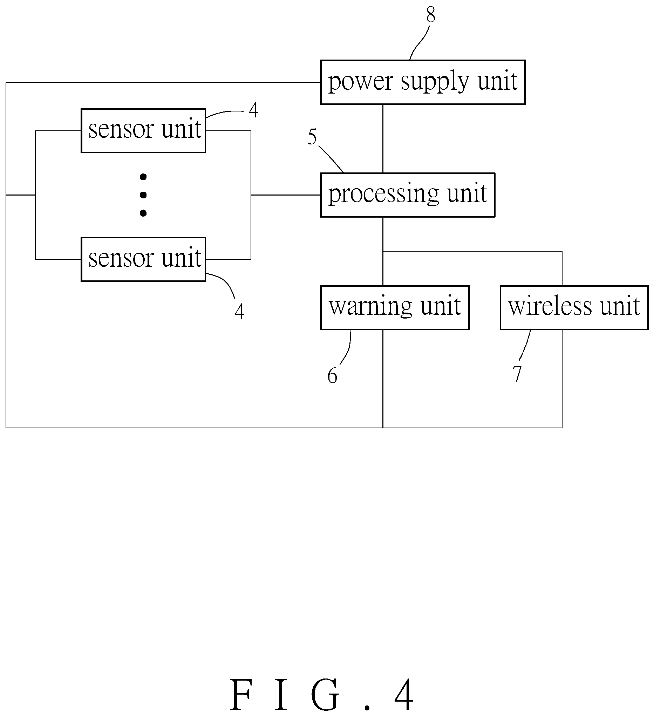

Referring to FIG. 4, a processing unit 5, a warning unit 6, a wireless unit 7 and a power supply unit 8 are provided in the ostomy body 1. The processing unit 5 is in signal communication with the sensor unit 4. The warning unit 6 and the wireless unit 7 are in signal communication with the processing unit 5, respectively. The power supply unit 8 is electrically connected to the sensor unit 4, the processing unit 5, the warning unit 6 and the wireless unit 7.

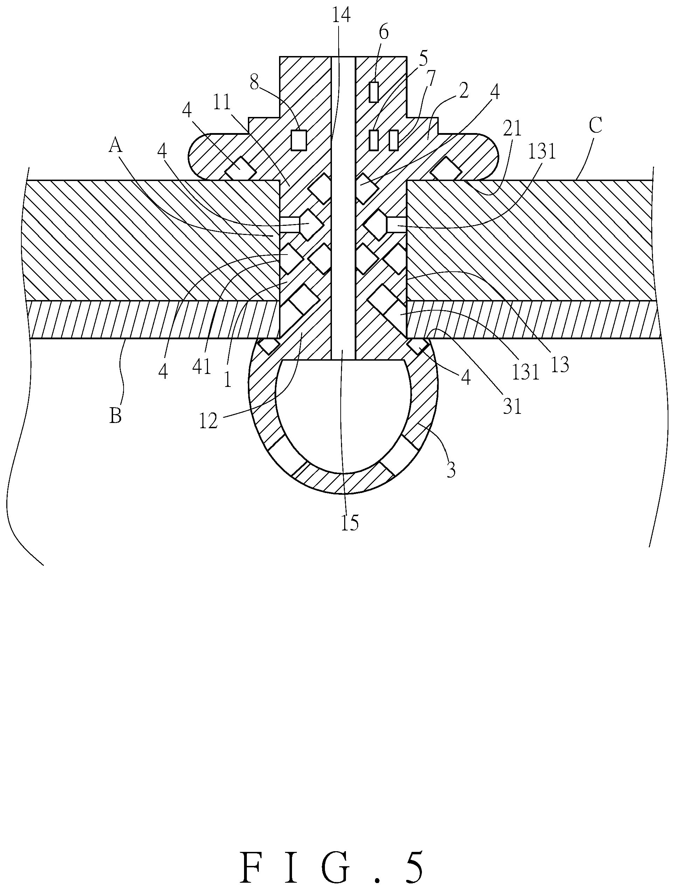

Referring to FIG. 5, the ostomy device of this embodiment is applied to, for example, a gastrostomy A. The inner retaining member 3 is located in the stomach, and the inner contact surface 31 is against the stomach wall B. The ostomy body 1 is located in the gastrostomy A, and the outer surface 13 is supported against the cut surface of the gastrostomy A. The outer retaining plate 2 is located on the body surface, and the outer contact surface 21 is against the skin C on the body surface.

The sensor unit 4 can continuously sense the condition of the gastrostomy A and transmit a sensor signal to the processing unit 5. When the sensor signal is abnormal, the processing unit 5 controls the warning unit 6 to issue a warning for reminding the caregiver to take care of the gastrostomy A. The processing unit 5 can transmit the sensor signal through the wireless unit 7 for remote care. The sensor unit 4, the processing unit 5, the warning unit 6 and the wireless unit 7, as shown in FIG. 5, are separate components. However, any two, any three or all of the sensing unit 4, the processing unit 5, the warning unit 6 and the wireless unit 7 may be integrated into a multi-function unit, which is also a feasible embodiment of the present invention.

In this embodiment, the sensor unit 4 may be a blood glucose sensor, a pH sensor, a tension sensor, a blood oxygen sensor, a temperature sensor, or a flow rate sensor. The blood glucose sensor and the blood oxygen sensor are, for example, arranged on the outer surface 13 of the ostomy body 1 for detecting the blood glucose level and blood oxygen level from the blood of the wound on cut surface of the gastrostomy A. The pH sensors are, for example, respectively arranged on the outer surface 13 and inner surface 14 of the ostomy body 1, the outer contact surface 21 of the outer retaining plate 2, and the inner contact surface 31 of the inner retaining member 3 for detecting whether there is a leak of digestive juice and the situation of the leak. The pH sensor may be arranged in the inner groove 131 of the outer surface 13. When little digestive juice is leaking and the pH sensors at other positions (flush with the outer surface 13, the inner surface 14, the outer contact surface 21 and the inner contact surface 31) don't detect the leak because the digestive juice runs quickly, the digestive juice accumulated in the inner groove 131 can be detected by the pH sensor in the inner groove 131, so as to achieve the purpose of real-time detection of the leak. The tension sensors may be arranged on the symmetrical positions of the outer surface 13 of the ostomy body 1, the outer contact surface 21 of the outer retaining plate 2, and the inner contact surface 31 of the inner retaining member 3, so that whether the ostomy device is correctly installed can be determined by the balance of pressure. The temperature sensor may be arranged at any position on the outer surface 13 and the inner surface 14 of the ostomy body 1, the outer contact surface 21 of the outer retaining plate 2, and the inner contact surface 31 of the inner retaining member 3 for sensing whether the body temperature around the gastrostomy A is normal. The flow rate sensor may be arranged on the outer surface 13 and the inner surface 14 of the ostomy body 1, the outer contact surface 21 of the outer retaining plate 2, and the inner contact surface 31 of the inner retaining member 3 for sensing the speed of digestive juice lead. The flow rate sensor arranged on the inner surface 14 is to sense the speed of tube-feeding.

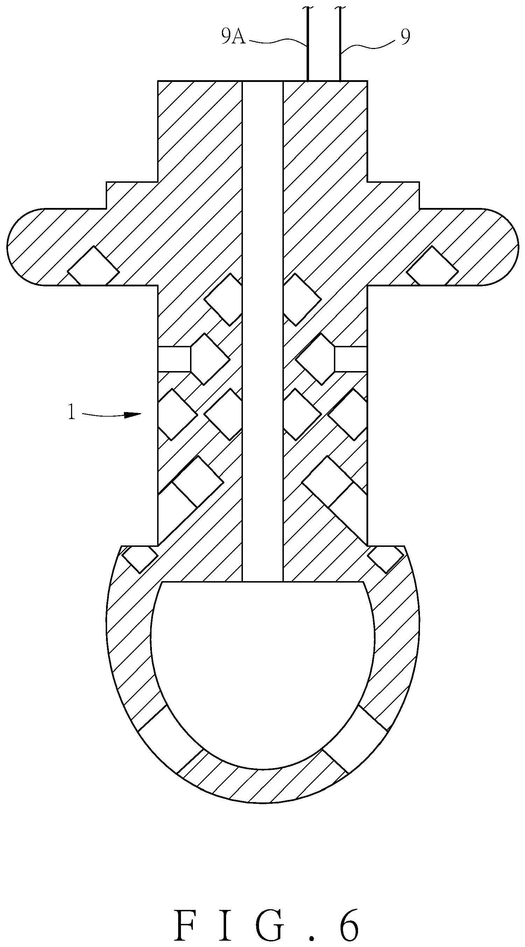

A second embodiment of the present invention, referring to FIG. 4 through FIG. 6, is substantially similar to the first embodiment with the exceptions described below. The processing unit 5, the warning unit 6, the wireless unit 7 and the power supply unit 8 are disposed on the outside of the ostomy body 1. The power supply unit 8 is connected to the sensor unit 4 with a wire 9. The processing unit 5 is connected to the sensor unit 4 through a signal wire 9A.

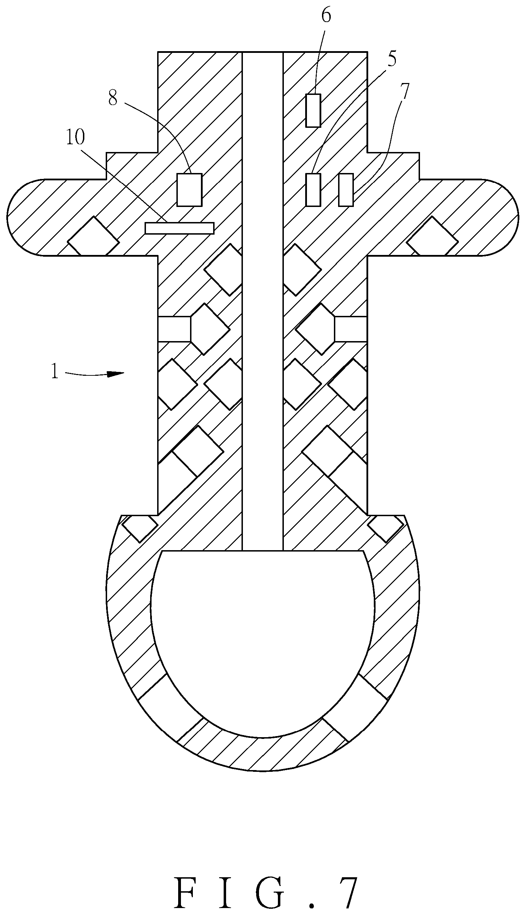

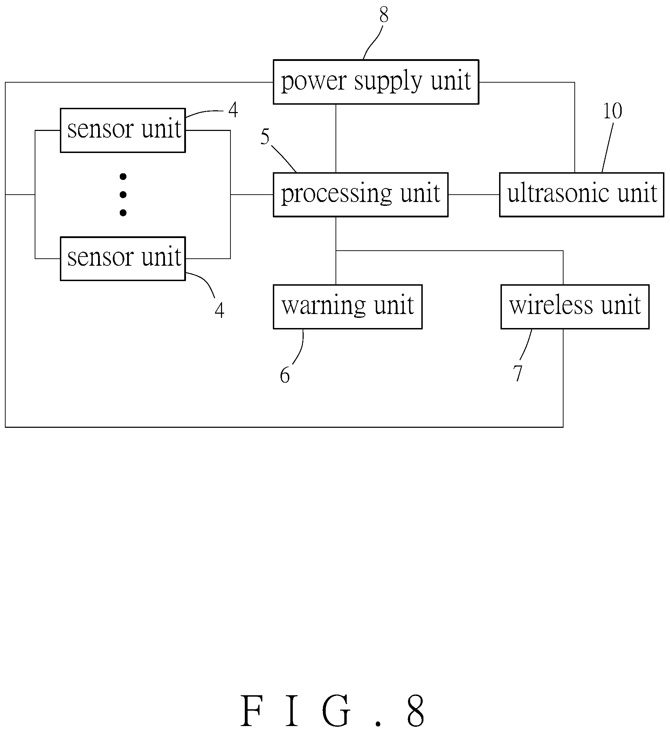

A third embodiment of the present invention, referring to FIG. 7 and FIG. 8, is substantially similar to the first embodiment with the exceptions described below. The ostomy device further comprises an ultrasonic unit 10 on the ostomy body 1. The ultrasonic unit 10 is electrically connected to the power supply unit 8 and is in signal communication with the processing unit 5. The ultrasonic unit 10 is configured to detect the suturing condition of the ostomy operation, whether the wound is purulent, and whether there is blood clot remaining in the stoma, and the like.

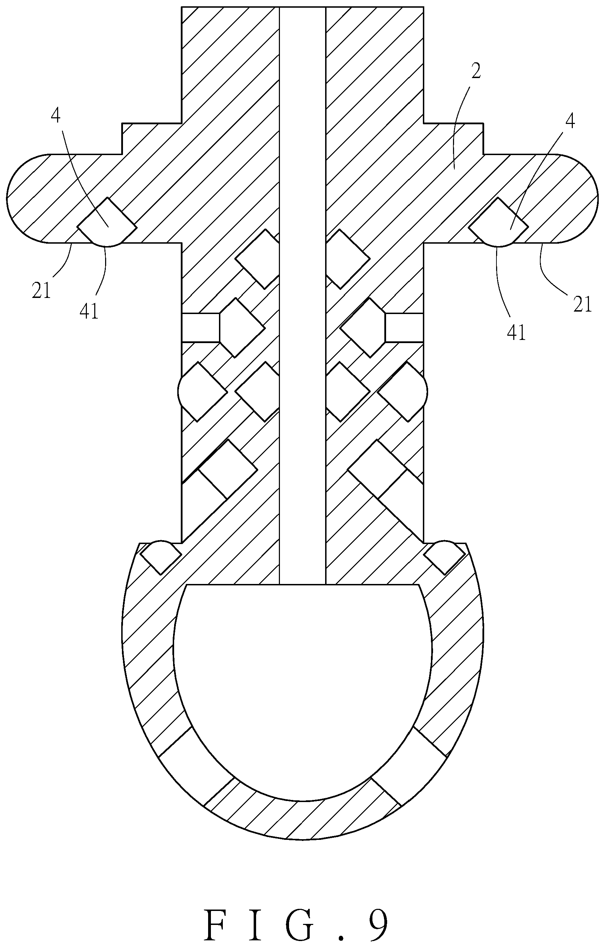

A fourth embodiment of the present invention, referring to FIG. 9, is substantially similar to the first embodiment with the exceptions described below. In this embodiment, the sensing surface 41 of the sensor unit 4 disposed on the outer contact surface 21 of the outer retaining plate 2 protrudes from the outer contact surface 21. Because the outer contact surface 21 is supported against the skin C on the body surface, the protruding sensing surface 41 will not cause discomfort, and can accurately detect whether the digestive juice leaks to the body surface.

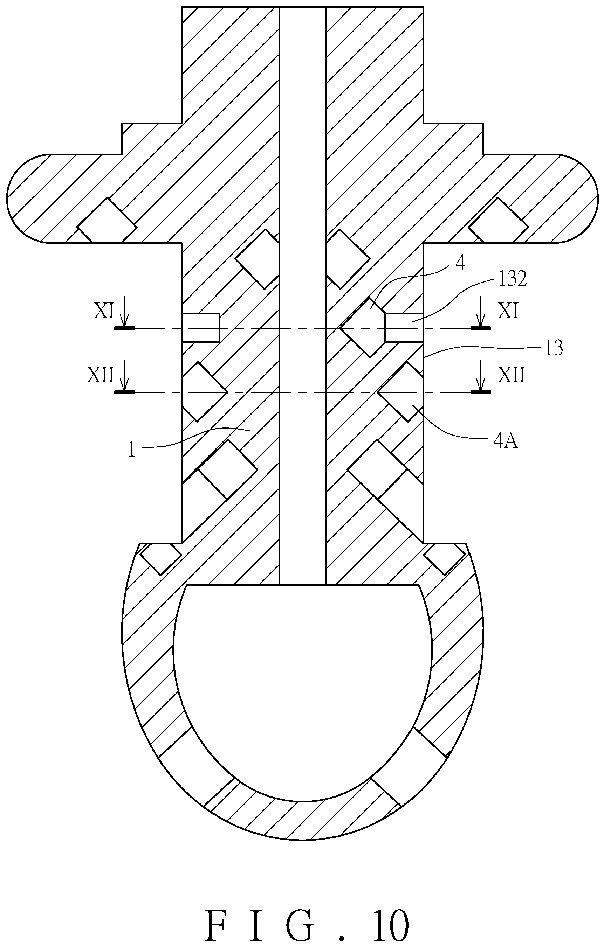

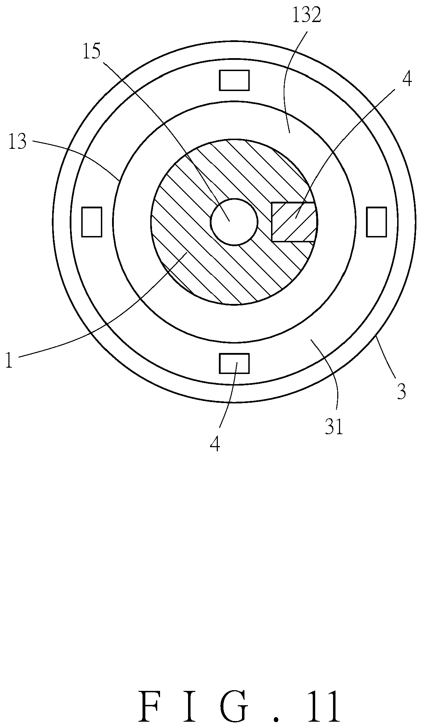

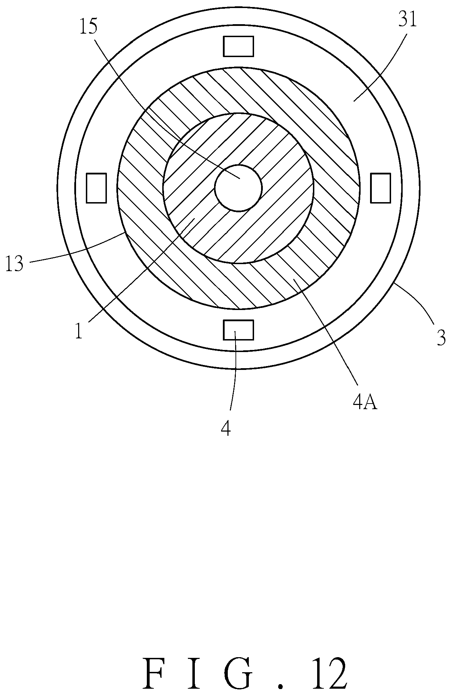

A fifth embodiment of the present invention, referring to FIG. 10 through FIG. 12, is substantially similar to the first embodiment with the exceptions described below. The outer surface 13 of the ostomy body 1 is formed with an annular inner groove 132. The sensor unit 4 is disposed in the annular inner groove 132. When the digestive juice leaks from the outer surface 13 of the ostomy body 1, the leaked digestive juice will accumulate in the annular inner groove 132, so that only one sensor unit 4 is able to sense a leak from all directions of the outer surface 13. As shown in FIG. 10 and FIG. 12, for example, an annular sensor unit 4A is provided on the outer surface 13 of the ostomy body 1, such that the conditions of the gastrostomy A in all directions can be sensed by the annular sensor unit 4A.

Although particular embodiments of the present invention have been described in detail for purposes of illustration, various modifications and enhancements may be made without departing from the scope of the present invention. Accordingly, the present invention is not to be limited except as by the appended claims.

## Claims

1. An ostomy device for digestive tracts, comprising:
an ostomy body (1), including a first end (11) and a second end (12), the ostomy body (1) further including an outer surface (13), an inner surface (14), and an ostomy hole (15) passing through the ostomy body (1);
an outer retaining plate (2), including an outer contact surface (21), the outer retaining plate (2) being disposed close to the first end (11);
an inner retaining member (3), including an inner contact surface (31), the inner retaining member (3) being disposed on the second end (12);
at least one sensor unit (4);
a processing unit (5), the processing unit (5) being in signal communication with the sensor unit (4) for receiving sensor information of the sensor unit (4);
a power supply unit (8), electrically connected to the sensor unit (4) and the processing unit (5);
**characterized by**:
the sensor unit (4) including a sensing surface (41), the sensor unit (4) being disposed on at least one of the outer surface (13), the inner surface (14), the outer contact surface (21) and the inner contact surface (31); the sensing surface (41) being exposed on the outer surface (13), the inner surface (14), the outer contact surface (21) or the inner contact surface (31).

2. The ostomy device of claim 1, wherein the sensor unit (4) is annularly arranged on the outer contact surface (21).

3. The ostomy device of claim 1, wherein the sensor unit (4) is annularly arranged on the inner contact surface (31).

4. The ostomy device of claim 1, wherein the sensor unit (4) is annularly arranged on the outer surface (13).

5. The ostomy device of claim 1, wherein the sensor unit (4) is annularly arranged on the inner surface (14).

6. The ostomy device of claim 1, wherein the sensor unit (4) is one of a blood glucose sensor, a pH sensor, a tension sensor, a blood oxygen sensor, a temperature sensor and a flow rate sensor.

7. The ostomy device of claim 1, further comprising an ultrasonic unit (10) on the ostomy body (1), wherein the ultrasonic unit (10) is electrically connected to the power supply unit (8) and is in signal communication with the processing unit (5).

8. The ostomy device of claim 1, further comprising a wireless unit (7), wherein the wireless unit (7) is in signal communication with the processing unit (5), the wireless unit (7) is electrically connected to the power supply unit (8), the wireless unit (7) and the processing unit (5) are disposed on the ostomy body (1), and the processing unit (5) receives a sensor signal from the sensor unit (4) and transmits the sensor signal through the wireless unit (7).

9. The ostomy device of claim 1, further comprising a warning unit (6), wherein the warning unit (6) is in signal communication with the processing unit (5), and the warning unit (6) is electrically connected to the power supply unit (8).

10. The ostomy device of claim 1, wherein the processing unit (5) and the power supply unit (8) are disposed on an outside of the ostomy body (1), the power supply unit (8) is connected to the sensor unit (4) with a wire (9), and the processing unit (5) is connected to the sensor unit (4) through a signal wire (9A).

11. The ostomy device of claim 1, wherein the sensing surface (41) is flush with or recessed in the outer contact surface (21), the inner surface (14), the outer surface (13), or the inner contact surface (31); or the sensing surface (41) protrudes from the outer contact surface (21).

12. The ostomy device of claim 1, wherein the sensor unit (4) is disposed in an inner groove (131).

13. The ostomy device of claim 12, wherein an extension axis of the inner groove (131) is perpendicular or inclined to the outer contact surface (21), the inner surface (14), the outer surface (13), or the inner contact surface (31).

14. The ostomy device of claim 12, wherein the inner groove (131) is annular.

15. The ostomy device of claim 1, wherein the sensor unit (4) is annular.

## Patentansprüche

1. Ostomievorrichtung für Verdauungstrakte, umfassend:
einen Ostomiekörper (1) mit einem ersten Ende (11) und einem zweiten Ende (12), wobei der Ostomiekörper (1) ferner eine Außenfläche (13), eine Innenfläche (14) und eine Ostomieöffnung (15) umfasst, die durch den Ostomiekörper (1) hindurch verläuft;
eine äußere Halteplatte (2) mit einer äußeren Kontaktfläche (21), wobei die äußere Halteplatte (2) in der Nähe des ersten Endes (11) angeordnet ist;
ein inneres Halteelement (3) mit einer inneren Kontaktfläche (31), wobei das innere Halteelement (3) an dem zweiten Ende (12) angeordnet ist;
mindestens eine Sensoreinheit (4);
eine Verarbeitungseinheit (5), die mit der Sensoreinheit (4) in Signalverbindung steht, um Sensorinformationen der Sensoreinheit (4) zu empfangen; und
eine Stromversorgungseinheit (8), die elektrisch mit der Sensoreinheit (4) und der Verarbeitungseinheit (5) verbunden ist;
wobei die Ostomievorrichtung **dadurch gekennzeichnet ist, dass**:
die Sensoreinheit (4) eine Erfassungsfläche (41) aufweist und auf mindestens einer der Außenfläche (13), der Innenfläche (14), der äußeren Kontaktfläche (21) oder der inneren Kontaktfläche (31) angeordnet ist, wobei die Erfassungsfläche (41) auf der Außenfläche (13), der Innenfläche (14), der äußeren Kontaktfläche (21) oder der inneren Kontaktfläche (31) freiliegt.

2. Ostomievorrichtung nach Anspruch 1, wobei die Sensoreinheit (4) ringförmig auf der äußeren Kontaktfläche (21) angeordnet ist.

3. Ostomievorrichtung nach Anspruch 1, wobei die Sensoreinheit (4) ringförmig auf der inneren Kontaktfläche (31) angeordnet ist.

4. Ostomievorrichtung nach Anspruch 1, wobei die Sensoreinheit (4) ringförmig auf der Außenfläche (13) angeordnet ist.

5. Ostomievorrichtung nach Anspruch 1, wobei die Sensoreinheit (4) ringförmig auf der Innenfläche (14) angeordnet ist.

6. Ostomievorrichtung nach Anspruch 1, wobei die Sensoreinheit (4) einer der folgenden Sensoren ist: ein Blutzuckersensor, ein pH-Sensor, ein Blutdrucksensor, ein Blutsauerstoff-Sensor, ein Temperatursensor oder ein Durchflusssensor.

7. Ostomievorrichtung nach Anspruch 1, ferner umfassend eine Ultraschalleinheit (10), die am Ostomiekörper (1) angeordnet ist, wobei die Ultraschalleinheit (10) elektrisch mit der Stromversorgungseinheit (8) verbunden ist und in Signalverbindung mit der Verarbeitungseinheit (5) steht.

8. Ostomievorrichtung nach Anspruch 1, ferner umfassend eine drahtlose Einheit (7), wobei die drahtlose Einheit (7) in Signalverbindung mit der Verarbeitungseinheit (5) steht, die drahtlose Einheit (7) elektrisch mit der Stromversorgungseinheit (8) verbunden ist, die drahtlose Einheit (7) und die Verarbeitungseinheit (5) am Ostomiekörper (1) angeordnet sind, und wobei die Verarbeitungseinheit (5) ein Sensorsignal von der Sensoreinheit (4) empfängt und dieses Sensorsignal über die drahtlose Einheit (7) überträgt.

9. Ostomievorrichtung nach Anspruch 1, ferner umfassend eine Warneinheit (6), wobei die Warneinheit (6) in Signalverbindung mit der Verarbeitungseinheit (5) steht und elektrisch mit der Stromversorgungseinheit (8) verbunden ist.

10. Ostomievorrichtung nach Anspruch 1, wobei die Verarbeitungseinheit (5) und die Stromversorgungseinheit (8) außerhalb des Ostomiekörpers (1) angeordnet sind, wobei die Stromversorgungseinheit (8) über einen Draht (9) mit der Sensoreinheit (4) verbunden ist und die Verarbeitungseinheit (5) über eine Signalleitung (9A) mit der Sensoreinheit (4) verbunden ist.

11. Ostomievorrichtung nach Anspruch 1, wobei die Erfassungsfläche (41) bündig mit oder vertieft in der äußeren Kontaktfläche (21), der Innenfläche (14), der Außenfläche (13) oder der inneren Kontaktfläche (31) angeordnet ist, oder die Erfassungsfläche (41) aus der äußeren Kontaktfläche (21) hervorsteht.

12. Ostomievorrichtung nach Anspruch 1, wobei die Sensoreinheit (4) in einer Innenrille (131) angeordnet ist.

13. Ostomievorrichtung nach Anspruch 12, wobei eine Verlängerungsachse der Innenrille (131) senkrecht oder geneigt zur äußeren Kontaktfläche (21), zur Innenfläche (14), zur Außenfläche (13) oder zur inneren Kontaktfläche (31) verläuft.

14. Ostomievorrichtung nach Anspruch 12, wobei die Innenrille (131) ringförmig ausgebildet ist.

15. Ostomievorrichtung nach Anspruch 1, wobei die Sensoreinheit (4) ringförmig ausgebildet ist.

## Revendications

1. Dispositif de stomie pour les voies digestives, **caractérisé par le fait qu'**il comprend :
un corps de stomie (1), comprenant une première extrémité (11) et une seconde extrémité (12), le corps de stomie (1) comprenant en outre une surface externe (13), une surface interne (14), et un trou de stomie (15) passant à travers le corps de stomie (1) ;
une plaque de retenue extérieure (2), comprenant une surface de contact externe (21), la plaque de retenue extérieure (2) étant disposée près de la première extrémité (11) ;
un élément de retenue intérieur (3), comprenant une surface de contact interne (31), l'élément de retenue intérieur (3) étant disposé sur la seconde extrémité (12) ;
au moins une unité de capteur (4) ;
une unité de traitement (5), l'unité de traitement (5) étant en communication par signaux avec l'unité de capteur (4) pour recevoir les informations du capteur de l'unité de capteur (4) ;
une unité d'alimentation électrique (8), raccordée électriquement à l'unité de capteur (4) et à l'unité de traitement (5) ;
**caractérisé par le fait que** :
l'unité de capteur (4) comprenant une surface de détection (41), l'unité de capteur (4) étant disposée sur au moins l'une des surface externe (13), surface interne (14), surface de contact externe (21) et surface de contact interne (31) ; la surface de détection (41) étant exposée sur la surface externe (13), la surface interne (14), la surface de contact externe (21) ou la surface de contact interne (31).

2. Dispositif de stomie selon la revendication 1, **caractérisé par le fait que** l'unité de capteur (4) est disposée de manière annulaire sur la surface de contact externe (21).

3. Dispositif de stomie selon la revendication 1, **caractérisé par le fait que** l'unité de capteur (4) est disposée de manière annulaire sur la surface de contact interne (31).

4. Dispositif de stomie selon la revendication 1, **caractérisé par le fait que** l'unité de capteur (4) est disposée de manière annulaire sur la surface externe (13).

5. Dispositif de stomie selon la revendication 1, **caractérisé par le fait que** l'unité de capteur (4) est disposée de manière annulaire sur la surface interne (14).

6. Dispositif de stomie selon la revendication 1, **caractérisé par le fait que** l'unité de capteur (4) est l'un des capteurs suivants : capteur de glycémie, capteur de pH, capteur de tension, capteur d'oxygène dans le sang, capteur de température et capteur de débit.

7. Dispositif de stomie selon la revendication 1, **caractérisé par le fait qu'**il comprend en outre une unité à ultrasons (10) sur le corps de stomie (1), l'unité à ultrasons (10) est raccordée électriquement à l'unité d'alimentation électrique (8) et est en communication par signaux avec l'unité de traitement (5).

8. Dispositif de stomie selon la revendication 1, **caractérisé par le fait qu'**il comprend en outre une unité sans fil (7), l'unité sans fil (7) est en communication par signaux avec l'unité de traitement (5), l'unité sans fil (7) est raccordée électriquement à l'unité d'alimentation électrique (8), l'unité sans fil (7) et l'unité de traitement (5) sont disposées sur le corps de stomie (1), et l'unité de traitement (5) reçoit un signal de capteur provenant de l'unité de capteur (4) et transmet le signal de capteur via l'unité sans fil (7).

9. Dispositif de stomie selon la revendication 1, **caractérisé par le fait qu'**il comprend en outre une unité d'avertissement (6), l'unité d'avertissement (6) est en communication par signaux avec l'unité de traitement (5), et l'unité d'avertissement (6) est raccordée électriquement à l'unité d'alimentation électrique (8).

10. Dispositif de stomie selon la revendication 1, **caractérisé par le fait que** l'unité de traitement (5) et l'unité d'alimentation électrique (8) sont disposées sur un extérieur du corps de stomie (1), l'unité d'alimentation électrique (8) est raccordée à l'unité de capteur (4) avec un fil (9), et l'unité de traitement (5) est raccordée à l'unité de capteur (4) par un fil de signalisation (9A).

11. Dispositif de stomie selon la revendication 1, **caractérisé par le fait que** la surface de détection (41) est affleurante ou encastrée dans la surface de contact externe (21), la surface interne (14), la surface externe (13), ou la surface de contact interne (31) ; ou la surface de détection (41) dépasse de la surface de contact externe (21).

12. Dispositif de stomie selon la revendication 1, **caractérisé par le fait que** l'unité de capteur (4) est disposée dans une rainure intérieure (131).

13. Dispositif de stomie selon la revendication 12, **caractérisé par le fait qu'**un axe de prolongement de la rainure intérieure (131) est perpendiculaire ou incliné vers la surface de contact externe (21), la surface interne (14), la surface externe (13), ou la surface de contact interne (31).

14. Dispositif de stomie selon la revendication 12, **caractérisé par le fait que** la rainure intérieure (131) est annulaire.

15. Dispositif de stomie selon la revendication 1, **caractérisé par le fait que** l'unité de capteur (4) est annulaire.
